(19) Europäisches Patentamt
European Patent Office
Office européen des brevets

(11) Veröffentlichungsnummer: **0 088 874**
**B1**

(12) # EUROPÄISCHE PATENTSCHRIFT

(45) Veröffentlichungstag der Patentschrift: **09.04.86**

(51) Int. Cl.⁴: **C 07 D 405/06**, A 61 K 31/41, A 61 K 31/415 // C07D307/28, C07C49/713

(21) Anmeldenummer: **83100898.2**

(22) Anmeldetag: **01.02.83**

(54) **Azolyl-phenoxy-tetrahydrofuran-2-yliden-methane, Verfahren zu ihrer Herstellung sowie antimikrobielle Mittel, die diese Stoffe enthalten.**

(30) Priorität: **11.02.82 DE 3204795**

(43) Veröffentlichungstag der Anmeldung: **21.09.83 Patentblatt 83/38**

(45) Bekanntmachung des Hinweises auf die Patenterteilung: **09.04.86 Patentblatt 86/15**

(84) Benannte Vertragsstaaten: **AT BE CH DE FR GB IT LI LU NL SE**

(56) Entgegenhaltungen: **EP - A - 0 035 087**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnoeckel 59, D-5600 Wuppertal 1 (DE)**
Erfinder: **Jautelat, Manfred, Dr., Muellersbaum 28, D-5093 Burscheid 1 (DE)**
Erfinder: **Büchel, Karl Heinz, Prof. Dr., Dabringhausener Strasse 42, D-5093 Burscheid (DE)**
Erfinder: **Schaller, Klaus, Dr., Bergerheide 47, D-5600 Wuppertal 1 (DE)**
Erfinder: **Plempel, Manfred, Dr., Zwengenberger Strasse 3c, D-5657 Haan (DE)**

**EP 0 088 874 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Azolyl-phenoxy-tetrahydrofuran-2-yliden-methane, ein Verfahren zu ihrer Herstellung sowie ihre Verwendung als antimikrobielle Mittel, insbesondere als Antimykotika.

Es ist bereits bekannt, geworden, daA bestimmte Azolylalkenole, wie z.B. im Phenoxyteil substituierte 1-(imi-dazol-1-yl)- bzw. -(1,2,4-triazol-1-yl)-2-phenoxy-4,4-dimethyl-1-penten-3-ole, gute antimykotische Eigenschaften aufweisen [vgl. DE-OS 29 28 968]. Die Wirkugn dieser Verbindungen ist jeodch nicht immer voll befriedigend.

Es wurden neue Azolyl-phenoxy-tetrahydrofuran-2-yliden-methane der allgemeinen Formel

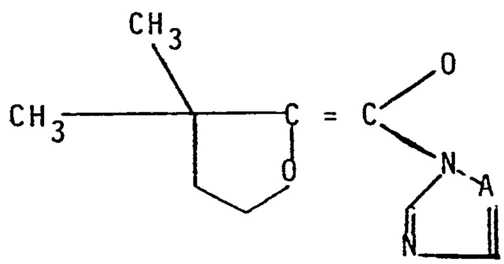

in welcher

A für ein Stickstoffatom oder die CH-Gruppe und

R für gegebenenfalls einfach bis dreifach, gleich oder verschieden substituiertes Phenyl, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffund 1 bis 5 gleichen oder verschiedenen Halogenatomen, wie Fluor- und Chloratomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy,

sowie deren physiologisch verträglichen Säureadditions-Salze gefunden.

Die Verbindungen der Formel (I) können in zwei geometrischen Isomerenformen vorliegen, je nach Anordnung der Gruppen, die an die Doppelbindung gebunden sind; vorzgusweise fallen sie in einem wechselnden Isomerenverhältnis an. Die vorliegende Erfindung betrifft sowohl die einzelnen Isomeren als auch die Isomeren-Gemische.

Bevorzugt sind diejenigen Verbindungen der Formel (I), in denen

A für ein Stickstoffatom oder die CH-Gruppe steht und

R für gegebenenfalls einfach bis dreifach, gleich oder verschieden substituiertes Phenyl steht, wobei als Substituenten genannt seien. Fluor, Chlor, Brom, Methyl, Ethyl, tert.-Butyl, Methoxy, Ethoxy, Methylthio, Trifluormethyl, Trifluormethoxy, Trifluormethylthio, Methoxycarbonyl, Ethoxycarbonyl, Nitro, Cyano, gegebenenfalls durch Chlor und/oder Methyl substituiertes Phenyl oder Phenoxy.

Weiterhin wurde gefunden, daß man die Azolyl-phenoxy-tetrahydrofuran-2-yliden-methane der Formel (I) erhält, wenn man Halogenetherketone der Formel

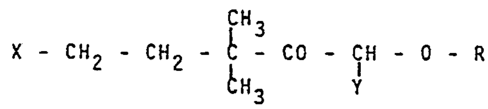
(II

in welcher

R die oben angegebene Bedeutung hat und

X und Y für Halogen stehen,

mit Imidazol oder 1,2,4-Triazol in Gegenwart eines Verdünnungsmittels und in Gegenwart eines Säurebindemittels umsetzt.

An die so erhaltenen Verbindungen der Formel (I) kann gegebenenfalls anschließend eine Säure addiert werden.

Die neuen Azolyl-phenoxy-tetrahydrofuran-2-yliden-methane der Formel (I) weisen starke antimykotische Eigenschaften auf. Dabei zeigen überraschenderweise die erfindungsge-mäßen Verbindungen eine allgemein bessere Wirksamkeit als die aus dem Stand der Technik bekannten, im Phenoxyteil substituierte, 1-(imidazol-1-yl)- bzw. -(1,2,4-Triazol-1-yl)-2-phenoxy-4,4-dimethyl-1-penten-3-ole, welches chemisch und wirkungsmäßig naheliegende Verbindungen sind. Die erfindungsgemäßen Stoffe stellen somit eine Bereicherung der Pharmazie dar.

Verwendet man beispielsweise 1-Brom-1-(4-chlorphenoxy)-5-chlor-3,3-dimethyl-2-pentanon und Imidazol als Ausgangsstoffe, so kann der Reaktionsablauf durch das folgende Formelschema wiedergegeben werden:

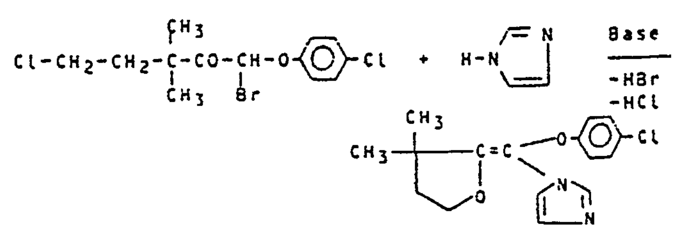


Die bei der Durchführung des erfindungsgemäßen Verfahrens als Ausgangsstoffe zu verwendenden Halogenetherketone sind durch die Formel (II) allgemein definiert. In dieser Formel steht R

vorzugsweise für die Reste, die bereits im Zusammenhang mit der Beschreibung der erfindungsgemäßen Stoffe der Formel (I) vorzugsweise für diesen Substituenten genannt wurden.

Die Halogenetherketone der Formel (II) sind noch nicht bekannt; sie können jedoch nach bekannten Verfahren hergestellt werden, indem man Halogenketone der Formel

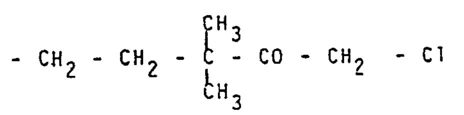

(III)

in welcher
X die oben angegebene Bedeutung hat,
mit bekannten Phenolen der Formel
H - O - R (IV)
in welcher
R die oben angegebene Bedeutung hat,
in üblicher Weise umsetzt und in den entstehenden Etherketonen der Formel

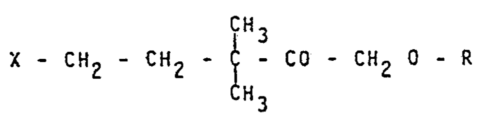

(V)

in welcher
R und X die oben angegebene Bedeutung haben,
das verbleibende aktive Wasserstoffatom in üblicher Weise gegen Halogen austauscht (vgl. auch die Herstellungsbeispiele). Die Halogenetherketone der Formel (II) können gegebenenfalls ohne Isolierung direkt weiter umgesetzt werden.

Die Halogenketone der Formel (III) sind ebenfalls nicht bekannt. Sie können gemäß einem eigenen Vorschlag [Europäisches Patent 87 596] erhalten werden, indem man 2-Chlormethylen-3,3-dimethyltetrahydrofuran der Formel

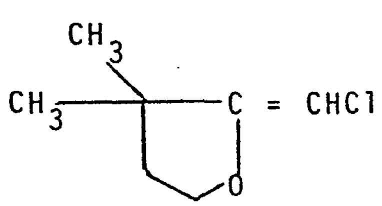

(VI)

mit aciden Verbindungen der Formel
H - X (VII)
in welcher
X die oben angegebene Bedeutung hat,
gegebenenfalls in Gegenwart eines inerten organischen Lösungsmittels, wie beispielsweise Toluol oder Methylenchlorid, bei Temperaturen zwischen 20 und 150 °C umsetzt.

Das 2-Chlormethylen-3,3-dimethyltetrahydrofuran der Formel (VI) ist ebenfalls noch nicht bekannt und ist ebenfalls Gegenstand eines eigenen Vorschlages [deutsche Offenlegungschrift DE-A 32 04 692]. Es wird durch sukzessive Umsetzung von 1,1,5-Trichlor-3,3-dimethyl-1-penten [vgl. europäische Patentanmeldung 45 426] mit Carboxylaten, wie beispielsweise Natriummethylat, in Gegenwart eines inerten organischen Lösungsmittels wie beispielsweise Dimethylformamid, unter Rückflußtemperatur erhalten.

Für die erfindungsgemäße Umsetzung kommen als Verdünnungsmittel inerte organische Lösungsmittel infrage. Hierzu gehören vorzugsweise Ketone, wie Diethylketon und insbesondere Aceton und Methylethylketon; Nitrile, wie propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, wie insbesondere Dimethylformamid; und halogenierte Kohlenwasserstoffe.

Die erfindungsgemäße Umsetzung wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin pyridin und Diazabicyclooctan.

Vorzugsweise verwendet man einen entsprechenden Überschuß an Azol.

Die Reaktionstemperaturen können bei der erfindungsgemäßen Umsetzung in einem größeren Bereich variiert werden. Im allgemeinen arbeitet man zwischen etwa 20 bis etwa 150°C, vorzugsweise bei 60 bis 120°C.

Bei der Durchführung der erfindungsgemäßen Umsetzung setzt man auf 1 Mol der Verbindungen der Formel (II) vorzugsweise 1 bis 4 Mol Azol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Die erfindungsgemäßen Verbindungen der Formel (I) können auch erhalten werden, wenn man

a) Halogenketone der Formel (III) mit Imidazol oder 1,2,4-Triazol gemäß den Bedingungen des erfindungs-gemäßen Verfahrens umsetzt, anschließend die so erhaltenen Azolyltetrahydrofuran-2-yliden-methane der Formel

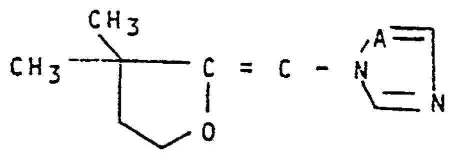

(VIII)

in welcher

A. die oben angegebene Bedeutung hat, zunächst mit Halogen, insbesondere mit Brom, und danach mit phenolen der Formel (IV) jeweils in üblicher Weise umsetzt;

b) das 2-Chlormethylen-3,3-dimethyltetrahydrofuran der Formel (VI) in üblicher Weise mit phenolen der Formel (IV) umsetzt, anschließend die so erhaltenen Phenoxy-tetrahydrofuran-2-yliden-methane der Formel

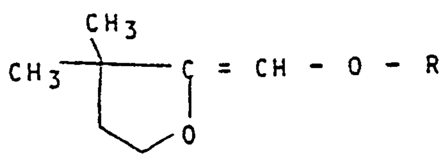

(IX)

in welcher

R die oben angegebene Bedeutung hat, zunächst in üblicher Weise mit Halogen, insbesondere mit Brom, und danach gemäß den Bedingungen des erfindungsge-mäßen Verfahrens mit Imidazol oder 1,2,4-Triazol umsetzt.

Die Azolyl-tetrahydrofuran-2-yliden-methane der Formel (VIII) und die Phenoxy-tetrahydrofuran-2-yliden-methane der Formel (IX) sind neu; sie stellen allgemein interessante Zwischenprodukte dar.

Zur Herstellung von physiologisch verträglichen Säureadditionssalzen der Verbindungen der Formel (I) kommen vorzugsweise folgende Säuren infrage: Die Halogenwasserstoffsäuren, z.B. die Chlorwasserstoffsäure und die Bromwasserstoffsäure, insbesondere die Chlorwasserstoffsäure, ferner Phosphorsäure, Salpetersäure, Schwefelsäure, monound bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Napthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wikungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosu, Aspergillus-Arten, wie Aspergillus niger und Aspergillus fumigatus, Trichophyton-Arten, Wie Trichophyton mentagrophytes, Microsporon-Arten, wie Microsporon felineum sowie Torulopsis-Arten, wie Torulopsis glabrate. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Draggés, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben,

Gele, Cremes, Lotions, Puder und Sprays genannt.

Tabletten, Dragees, Kapseln, Pilien und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

**Herstellungsbeispiele**

**Beispiel 1**

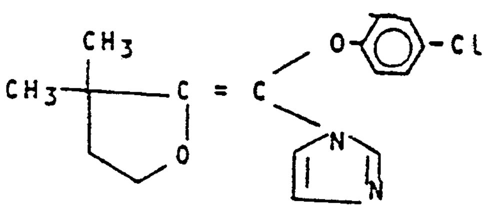

35 g (0,5 Mol) Imidazol und 70 g (0,5 Mol) Kaliumcarbonat werden in 700 ml Toluol gelöst. Diese Mischung wird bei 80° C mit 93 g (0,26 Mol) 1-Brom-1-(4-chlorphenoxy)-5-chlor-3,3-dimethyl-2-pentanon in 200 ml Toluol versetzt. Man läßt das Reaktionsgemisch 10 Stunden bei 90° C nachrühren, kühlt und saugt vom anorganischen Rückstand ab. Das Filtrat wird mit Wasser gewaschen, über Natriumsulfat getrocknet und eingeengt. Der Rückstand wird säulenchromatographisch (Kieselgel: Essigester/Cyclohexan = 3/1) gereinigt. Man erhält 12,6 g (14,2 % der Theorie) (4-Chlorphenoxy)-(imidazol-1-yl)-3,3-dimethyltetrahydrofu-ran-2-yliden-methan vom Schmelzpunkt 85 - 88° C.

**Herstellung des Ausgangsproduktes**

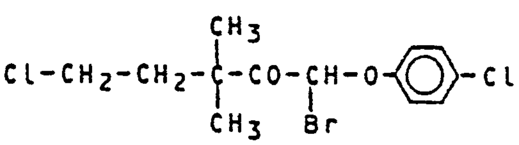

136 g (0,5 Mol) 1-(4-Chlorphenoxy)-5-chlor-3,3-dimethyl-2-pentanon werden in 1000 ml Methylenchlorid gelöst. Man läßt bei Raumtemperatur 79,9g (1 Mol) Brom so zutropfen, daß sich die Lösung immer entfärbt. Anschließend wird 1 Stunde bei Raumtemperatur nachgerührt und das Reaktionsgemisch durch Abdestillieren des Lösungsmittels eingeengt. Man erhält quantitativ, d.h. 177 g, 1-Brom-1-(4-chlor-phenoxy)-5-chlor-3,3-dimethyl-2-pentanon, das direkt weiter umgesetzt wird.

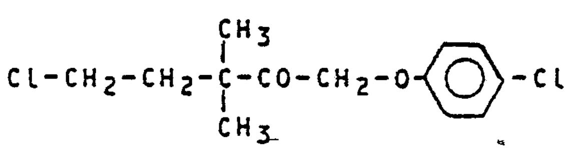

92,5 g (0,72 Mol) 4-Chlorphenol und 99,4 g (0,72 Mol) Kaliumcarbonat werden 2 Stunden in 500 ml Toluol unter Rückfluß erhitzt, wobei das Reaktionswasser azeotrop abdestilliert. Man kühlt auf 40° C ab und versetzt mit 110 g (0,6 Mol) 1,5-Dichlor-3,3-dimethyl-2-pentanon in 300 ml Toluol. Das Reaktionsgemisch wird 5 Stunden auf 100° C erhitzt, danach abgekühlt und vom anorganischen Rückstand abgesaugt. Das Filtrat wird mit verdünnter Natronlauge und Wasser gewaschen, über Natriumsulfat getrocknet und eingeengt. Man erhält 136,3 g (82,6 % der Theorie) rohes 1-(4-Chlorphenoxy)-5-chlor-3,3-dimethyl-2-pentanon, das direkt weiter umgesetzt wird.

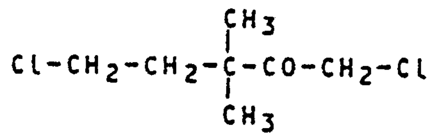

In 476 g (3,25 Mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert und die Innentemperatur steigt bis auf 30° C. Nach vollständiger Sättigung mit Chlorwasserstoff wird das Reaktionsgemisch 2 Stunden bei Raumtemperatur nachgerührt. Überschüssiger Chlorwasserstoff wird zunächst in die Wasserstrahlpumpe gezogen, dann wird bei gutem Vakuum destilliert. Man erhält 531 g (90 % der Theorie) 1,5-Dichlor-3,3-dimethyl-2-pentanon vom Siedepunkt 85 - 90° C/0,3 mbar.

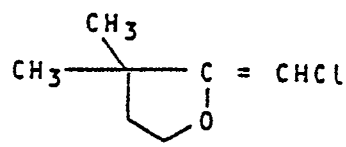

806 g (4 Mol) 1,1,5-Trichlor-3,3-dimethyl-1-penten werden mit 360 g (4,4 Mol) wasserfreiem Natriumacetat in 1 l Dimethylformamid 6 Stunden unter Rückfluß erhitzt. Nach Abkühlen auf ca. 100° C tropft man 1,6 l (8 Mol) 30 %-ige Natriummethylatlösung in Methanol ein und erhitzt weitere 4 Stunden unter Rückfluß. Die kalte Lösung wird in Wasser gegossen und mit Methylenchlorid mehrfach extrahiert.

Nach Trocknen der Lösung und Abdestillieren des Lösungsmittels bleiben 654 g Produkt zurück, das über eine Kolonne fraktioniert wird. Man erhält 522 g (89 % der Theorie) 2-Chlormethylen-3,3-dimethyltetrahydrofuran vom Siedepunkt 84 - 87° C/20 mbar.

Analog werden die folgenden Verbindungen der allgemeinen Formel (I)

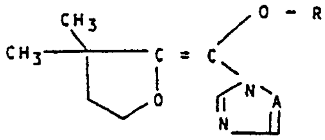 (I)

erhalten:

| Bsp.Nr. | R | A | Schmelzpunkt(°C) bzw. $n_D^{20}$ |
|---|---|---|---|
| 2 | 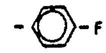 | CH | 214 |
| 3 | 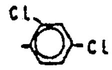 | CH | 119(A-Form) |
| 4 | 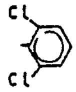 | CH | 82 |
| 5 | 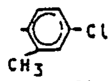 | CH | 109(A-Form) |
| 6 | 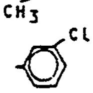 | CH | 1,5607 |
| 7 | 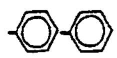 | CH | 95-105 |
| 8 | 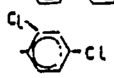 | CH | 95-105 |
| 9 | 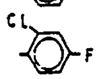 | CH | Kristall-masse |
| 10 | 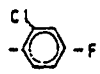 | CH | 118(A-Form) |
| 11 | 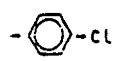 | N | 124 |
| 12 | 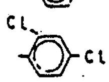 | N | zähes Oel |
| 13 | 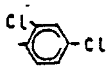 | N | 115-20(A-Form) |
| 14 | 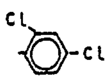 | N | 1,5578(B-Form) |
| 15 | 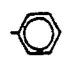 | CH | 85-90 |

A- und B-Form: die beiden möglichen Isomerenformen

**Verwendungsbeispiele**

In dem nachfolgenden Beispiel werden die nachstehend angegebenenen Verbindungen als Vergleichssubstanzen eingesetzt:

(A) 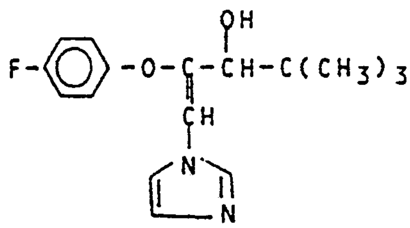

(B) 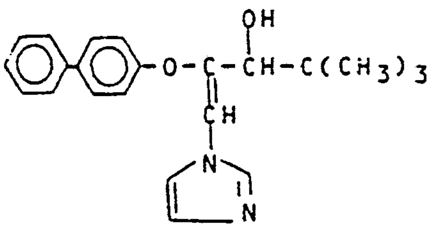

(C) 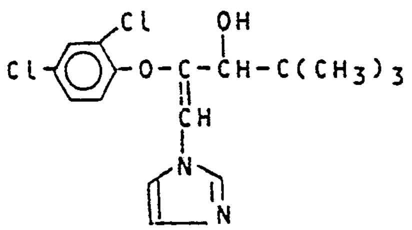

(D) 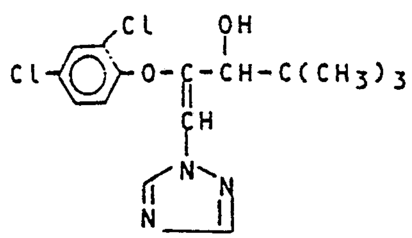

**Beispiel A**

**Antimykotische in-vitro-Wirksamkeit**

Versuchsbeschreibung:

Die in-vitro-Prüfungen wurden im Reihenverdünnungstest mit Keiminokula von durchschnittlich 5 x $10^4$ Keimen/ml Substrat durchgeführt. Als Nährmedium dienten

a) für Dermatophyten und Schimmelpilze: Sabourand's milieu d'épreuve

b) für Hefen: Fleischextrakt-Traubenzucker-Boullion.

Die Bebrütungstemperatur betrug 20°C, die Bebrütungsdauer lag bei 24 bis 98 Stunden bei Hefen und 98 8tunden bei Dermetophyten und Schimmelpilzen.

In diesem Test zeigen insbesondere die Verbindungen der Beispiele 1, 2, 3, 4, 5, 6, 7, 8 und 11 eine bessere antimykotische Wirkung als die aus dem Stand der Technik bekannten Verbindungen (A), (B), (C) und (D).

**Tabelle A**

Tabelle A

Antimykotische in-vitro-Wirksamkeit

| Wirkstoff | MHK-Werte in γ/ml Nährmediu | | | |
|---|---|---|---|---|
| | Trichophyton mentagr. | Microsporum canis | Candida albicans | Torulopsis glabrata |
| (A) (bekannt) | 1 | 64 | 32 | 64 |
| (B) (bekannt) | <1 | 32 | 32 | >64 |
| (C) (bekannt) | <1 | 32 | 16 | 16 |
| (D) (bekannt) | <1 | 64 | 32 | 32 |
| gemäß Herst.Bsp. | | | | |
| 1 | <1 | <1 | 8 | 2 |
| 2 | <1 | <1 | <1 | 2 |
| 3 | <1 | <1 | <1 | <1 |
| 4 | <1 | <1 | 16 | 16 |
| 5 | <1 | <1 | <1 | <1 |
| 6 | <1 | <1 | 16 | <1 |
| 7 | <1 | <1 | <1 | <1 |
| 8 | <1 | <1 | <1 | <1 |
| 11 | <1 | 16 | 4 | 16 |

**Patentansprüche**

1. Azolyl-phenoxy-tetrahydrofuran-2-yliden-methane der allgemeinen Formel

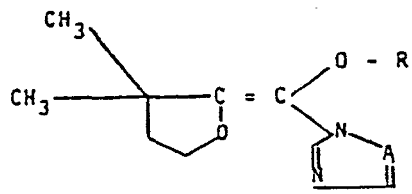

(I)

in welcher

A für ein Stickstoffatom oder die CH-Gruppe und

R für gegebenenfalls einfach bis dreifach, gleich oder verschieden substituiertes Phenyl, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Fluor- und Chloratomen, Alkoxycarbonyl mit 1 bis 4 Kohlenstoffatomen im Alkylteil, Mitro, Cyano oder gegebenenfalls durch Halogen und/oder Alkyl mit 1 bis 2 Kohlenstoffatomen substituiertes Phenyl oder Phenoxy.

2. Azolyl-phenoxy-tetrahydrofuran-2-yliden-methane der allgemeinen Formel (I) in Anspruch 1, in welcher

A für ein Stickstoffatom oder die CH-Gruppe steht und

R für gegebenenfalls einfach bis dreifach, gleich oder verschieden substituiertes Phenyl steht, wobei als

Substituenten genannt seien: Fluor, Chlor, Brom, Methyl, Ethyl, tert.-Butyl, Methoxy, Ethoxy, Methylthio, Trifluormethyl, Trifluormethoxy, Trifluormethylthio, Methoxycarbonyl, Ethoxycarbonyl, Nitro, Cyano, gegebenenfalls durch Chlor und/oder Methyl substituiertes Phenyl oder Phenoxy.

3. (4-Chlorphenoxy)-(imidazol-1-yl)-3,3-dimethyl-tetra-hydrofuran-2-yliden-methan.

4. (4-Fluorphenoxy)-(1-imidazol-1-yl)-3,3-dimethyl-tetra-hydrofuran-2-yliden-methan.

5. (2,4-Dichlorphenoxy)-(imidazol-1-yl)-3,3-dimethyl-tetra-hydrofuran-2-yliden-methan.

6. Verfahren zur Herstellung von Azolyl-phenoxy-tetra-hydrofuran-2-yliden-methane der Formel (I) in Anspruch 1, dadurch gekennzeichnet, daA man Halogenetherketone der Formel

X - $CH_2$ - CH-

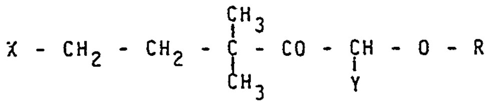

(II)

in welcher

R die in Anspruch 1 angegebene Bedeutung hat und

X und Y für Halogen stehen,

mit Imidazol oder 1,2,4-Triazol in Gegenwart eines Verdünnungsmittels und in Gegenwart eines Säurebindemittels umsetzt.

7. Arzneimittel, gekennzeichnet durch einen Gehalt an mindestens einem Azolyl-phenoxy-tetrahydrofuran-2-yliden-methan gemäß Anspruch 1.

8. Verfahren zur Herstellung von antimykotischen Mitteln, dadurch gekennzeichnet, daß man ein Azolylphenoxy-tetrahydrofuran-2-yliden-methan gemäß Anspruch 1 mit inerten, nichttoxischen, pharmazeutisch geeigneten Trägerstoffen vermischt.

**Claims**

1. Azolyl-phenoxy-tetrahydrofuran-2-ylidene-methanes of the general formula

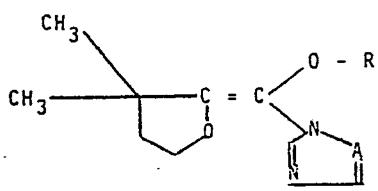

(I)

in which

A a nitrogen atom or the CH group and R phenyl which is optionally mono- to trisubstituted by identical or different substituents, the following being mentioned as substituents: halogen, alkyl having 1 to 2 carbon atoms alkoxy and alkylthio each having 1 to 2 carbon atoms halogenoalkyl, halogenoalkoxy and halogenoalkylthio each having 1 to 2 carbon and 1 to 5 identical or different fluorine and chlorine atoms, alkoxycarbonyl having 1 to 4 carbon atoms in the alkyl part, nitro, cyano or phenoxy or phenyl optionally substituted by halogen and/or alkyl having 1 to 2 carbon atoms.

2. Azolyl-phenoxy-tetrahydrofuran-2-ylidene-methanes of the general formula (I) in Claim 1, in which

A represents a nitrogen atom or the CH group and R represents phenyl which is optionally mono- to trisubstituted by identical or different substituents, the following being mentioned as substituents: fluorine, chlorine, bromine, methyl, ethyl, tert.-butyl, methoxy, ethoxy, methylthio, trifluoromethyl, trifluoromethoxy, trifluoromethylthio, methoxycarbonyl, ethoxycarbonyl, nitro, cyano or phenoxy or phenyl optionally substituted by chlorine and/or methyl.

3. (4-Chlorophenoxy)-(imidazol-1-yl)-3,3-dimethyl-tetrahydro-furan-2-ylidene-methane.

4. (4-Fluorophenoxy)-(1-imidazol-1-yl)-3,3-dimethyl-tetra-hydrofuran-2-ylidene-methane.

5. (2,4-Dichlorophenoxy)-(imidazol-1-yl)-3,.-dimethyl-tetrahydrofuran-2-ylidene-methane.

6. Process for the preparation of azolyl-phenoxy-tetrahydrofuran-2-ylidene-methanes of the formula (I) in Claim 1, characterised in that halogeno-ether-ketones of the formula

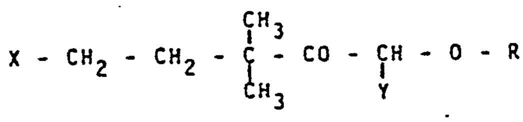

in which

R has the meaning given in Claim 1 and

X and Y represent halogen,

are reacted with imidazole or 1,2,4-triazole in the presence of a diluent and in the presence of an acid-binding agent.

7. Medicaments, characterised in that they contain at least one azolyl-phenoxy-tetrahydrofuran-2-ylidene-methane according to Claim 1.

8. process for the preparation of antimycotic agents, characterised in that an azolylphenoxy-tetrahydrofuran-2-ylidene-methane according to Claim 1 is mixed with inert, non-toxic, pharmaceutically suitable excipients.

**Revendications**

1. Azolyl-phenoxy -tetrahydrofuran-2-ylidène-méthanes de formule générale:

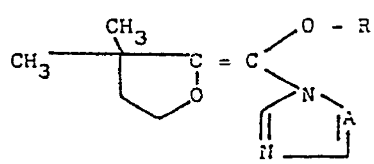

(I)

dans laquelle

A représente un atome d'azote ou le groupe CH, et

R représente un groupe phényle éventuellement substitué une à trois fois de manière identique ou différente en mentionnant,

comme substituants: les atomes d'halogènes, les groupes alkyle contenant 1 à 4 atomes de carbone, les groupes alcoxy et les groupes alkylthio contenant chacun 1 ou 2 atomes de carbone, les groupes halogénalkyle, les groupes halogénalcoxy et les groupes halogénalkylthio contenant chacun 1 ou 2 atomes de carbone et 1 à 5 atomes d'halogènes identiques ou différents tels que les atomes de fluor et de chlore, les groupes alcoxycarbonyle contenant 1 à 4 atomes de carbone dans la fraction alkyle, le groupe nitro, le groupe cyano ou le groupe phényle ou le groupe phénoxy éventuellement substitué par un atome d'halogène et/ou par un groupe alkyle contenant 1 ou 2 atomes de carbone.

2. Azolyl-phénoxy-tétrahydrofuran-2-ylidène-méthanes de formule générale (I) selon la revendication 1, formule dans laquelle

A représente un atome d'azote ou le groupe CH et

R représente un groupe phényle éventuellement substitué une à trois fois de manière identique ou différente en mentionnant, comme substituants: l'atome de fluor, l'atome de chlore, l'atome de brome, le groupe méthyle, le groupe éthyle, le groupe tert-butyle, le groupe méthoxy, le groupe éthoxy, le groupe méthylthio, le groupe trifluorométhyle, le groupe trifluorométhoxy, le groupe trifluorométhylthio, le groupe méthoxycarbonyle, le groupe éthoxycarbonyle, le groupe nitro, le groupe cyano, le groupe phényle ou le groupe phénoxy éventuellement substitué par l'atome de chlore et/ou le groupe méthyle.

3. (4-chlorophénoxy)-(imidazol-1-yl)-3,5-diméthyl-tétrahydrofuran-2-ylidène-méthane.

4. (4-fluorophénoxy)-(1-imidazol-1-yl)-3,3-diméthyl-tétrahydrofuran-2-ylidène-méthane.

5. (2,4-dichlorophénoxy)-(imidazol-1-yl)-3,3-diméthyl-tétrahydrofuran-2-ylidène-méthane.

6. Procédé de préparation d'azolyl-phénoxy-tétrahydrofuran-2-ylidène-méthanes de formule (I) selon la revendication 1, caractérisé en ce qu'on fait réagir des halogéno-éther-cétones de formule:

$$X - CH_2 - CH_2 - \underset{CH_3}{\overset{CH_3}{C}} - CO - \underset{Y}{CH} - O - R \qquad (II)$$

dans laquelle

R a la signification indiquée dans la revendication 1, et

X et Y représentent chacun un atome d'halogène, avec l'imidazole ou le 1,2,4-triazole en présence d'un diluant et en présence d'un agent fixateur d'acide.

7. Médicaments, caractérisés en ce qu'ils contiennent au moins un azolyl-phénoxy-tétrahydro-furan-2-ylidène-méthane selon la revendication 1.

8. Procédé de préparation d'agents antimycotiques, caractérisé en ce qu'on mélange un azolyl-phénoxy-tétrahydrofuran-2-ylidène-méthane selon la revendication 1 avec des substances supports inertes, non toxiques et pharmaceutiquement appropriées.